# EUROPEAN PATENT APPLICATION

(11) **EP 3 421 615 A1**
(43) Date of publication of application: **02.01.2019**
(21) Application number: 18178027.1
(22) Date of filing: 29.03.2013
(51) Int. Cl.: C12Q 1/6883

(54) **METHOD AND KIT FOR THE CLASSIFICATION AND PROGNOSIS OF TISSUE OR ORGAN IN A REPARATIVE OR REACTIVE PROCESS**

(30) Priority: 30.03.2012 WO PCT/IB2012/000906
(62) Divisional of application: 13713192.6
(71) Applicant: Urgo Recherche Innovation et Développement, 21300 Chenove (FR); Centre National de la Recherche Scientifique CNRS, 75794 Paris Cedex 16 (FR); Université Paris Diderot - Paris 7, 75013 Paris (FR); École Normale Supérieure, 75005 Paris (FR)
(72) Inventor: DUGAST DARZACQ, Claire, Berkeley 94707 California (US); DARZACQ, Xavier, 92160 Antony (FR); NOIZET, Maïté, 31300 Toulouse (FR); LAGOUTTE, Emilie, 94270 Le Kremlin Bicetre (FR); ROEST CROLLIUS, Hugues, 94160 Saint Mande (FR); GRATIGNY, Marlène, 92700 Colombes (FR); BOUSCHBACHER, Marielle, 21220 Chambolle-Musigny (FR)
(74) Representative: Cabinet Plasseraud

(57) **Abstract**

The invention relates to methods of diagnosis or prognosis of a tissue or organ susceptible of developing a fibrosis, such as a lung or skin fibrosis, comprising the step of determining the levels of expression of genes, e.g. PI16, encoding different molecular markers in a sample of a tissue or organ from a mammalian, wherein said genes show differential expression when compared with the expression in normal fibroblasts of said mammalian.

## Description

### Field of the invention

The present invention relates to a method and kit for the classification and prognosis of organ or tissue in a reparative or reactive process in order to determine whether the tissue or organ may develop a fibrosis or not. The method defines a molecular signature that enables one to characterize a pathological organ or tissue being in a reparative or reactive process. Preferably, the present invention relates to a method and kit for the classification and prognosis of wounds of mammalian, in particular in human. The method defines a molecular signature that enables one to characterize a pathological wound healing leading to an abnormal scar, or a fibrosis (hypertrophic scars or keloids).

### Background of the invention

Fibrosis is the formation of excess fibrous tissues or scar tissue in an organ or a tissue. Fibrosis is a common pathophysiological response of tissues to chronic injury or long-term inflammation. There are many potential origins of this fibrosis. It can be induced by a disease (inherited or not), by side effects of a treatment (for example radiation or chemotherapy), by a toxic environment (for example smoking), or by an injury. It can affect different organs such as the skin or the lung.

Fibrosis induced generally the failure of the tissue of the organ that is affected. Fibrotic tissue is like a scar tissue, stiff, thick, and rigid. Sometimes, it can also swell. For example, in the lung, fibrosis lead to a shortness of breath particularly during exercise and a dry and hacking cough, due to the abnormal expansion of the fibrosis lung.

Some examples of fibrosis are pulmonary fibrosis (lung), cystic fibrosis (lung and digestive system), Crohn's disease (intestine), scleroderma/systemic sclerosis (lung or skin), arthrofibrosis (knee, shoulder, other joints), cutaneous fibrosis with hypertrophic or keloid scars

When the tissue concerned by this phenomenon is the skin, it is principally the wound healing process that is affected.

The natural wound healing is divided into three sequential phases; each phase is characterized by specific cellular activities: the inflammatory phase, the proliferative phase and the remodeling phase.

The first phase, called the inflammatory phase, begins minutes after injury. The blood vessels rupture induces the clot formation, composed mainly of fibrin and fibronectin. The clot fills partially the lesion and allows the migration of the inflammatory cells within the lesion. The inflammatory cells are recruited to debride the wound. Platelets secrete factors, such as growth factors or cytokines, which induce the recruitment of cells implicated in the wound healing (inflammatory cells such as neutrophils and macrophages, fibroblasts and endothelial cells).

The second phase is called the proliferative phase and corresponds to the development of the granulation tissue. Fibroblasts migrate into the wound area, proliferate and form a new provisional extracellular matrix by secreting extracellular matrix (ECM) proteins. Then endothelial cells migrate to promote the neovascularization or angiogenesis of the lesion. Inside the granulation tissue, fibroblasts activate and differentiate into myofibroblasts, presenting contractile properties thanks to their expression of alpha-smooth muscle actin (similar to that in smooth muscle cells). Myofibroblasts have a key role in wound healing as they provide the contraction of the wound. Finally, keratinocytes migrate from the wound edge, proliferate and differentiate to reconstitute the epidermis.

The last phase of the wound healing process appears after the wound closure. It corresponds to the remodeling of the granulation tissue. The granulation tissue is reorganized, type III collagen is replaced by type I collagen, as normal dermis is principally composed of type I collagen. During this phase, myofibroblasts in excess are eliminated by apoptosis. The last phase of the wound healing is long. One year after injury, the scar is remodeled; it gets less red and thinner.

However, this process is not only complex but fragile; it is susceptible to interruption or failure leading to the formation of chronic or non-healing wounds or formation of abnormal scars. Factors which may contribute to this include diseases (such as diabetes, venous or arterial disease), age, infection or tissue localization.

Fibrosis, hypertrophic scars and keloids

Fibrosis is a common pathophysiological response of tissues to chronic injury. Fibrosis affects different organs such as the skin or the lung. Fibrosis is characterized by a differentiation of fibroblasts into myofibroblasts and an excessive accumulation of connective tissue. Fibrosis induces a loss of function of the organ and potentially the failure of the organ.

Hypertrophic, keloid or fibrous scars result from abnormal wound healing. These scars are characterized by an excessive deposit of ECM proteins, especially collagen. In these abnormal wounds, granulation tissue is hyper proliferative, due to an excess of myofibroblasts (Armour A, Scott PG, Tredget EE. Wound Repair Regen. 2007 Sep-Oct;15 Suppl 1:S6-17. Review. Erratum in: Wound Repair Regen. 2008 Jul-Aug;16(4):582).

In normal wound healing, fibroblasts get activated, and then differentiate into myofibroblasts presenting contractile properties thanks to their expression of alpha-smooth muscle actin (αSMA). Myofibroblasts are responsible for the deposit of extra cellular matrix and for the wound closure by moving closer the wound edges. In hypertrophic scar, keloid or fibrous wound healing, the activity of myofibroblasts persists and leads to tissue deformation, which is particularly evident, for example, in hypertrophic scars developed after burn injury.

Hypertrophic and keloid scars are characterized by deposit of excessive amounts of collagen leading to a raised scar (more intense in keloids than in hypertrophic scars). They are formed most often at the sites of pimples, cuts and burns.

Some hypertrophic scars are non-functional scars as they limit the function of the skin where they developed. They generate a loss of mobility of the scar zone and the neighboring zones, which can completely limit the movements (for example, elbow and mobility of the arm). They are mostly the result of burns of specific anatomical zones.

In some pathological diseases or specific anatomic localizations, early diagnosis of the potential onset of a wound may help to prevent the development of an abnormal scar or a skin fibrosis. In the situation where a wound or a skin fibrosis has already developed, knowledge of the diagnosis or prognosis of a wound may enable patients to receive maximum benefit from therapy. For fibrosis, hypertrophic scars or keloids, there are no techniques available for the prediction of these disorders. It is known that some phototypes or tissue localization, such as joints, are more likely to develop keloids or hypertrophic scars, but no reliable prognosis or diagnosis method is known.

However, there remains a need in the art for a method for the early diagnosis or prognosis of wound fate. In particular, there is a need for a sensitive and reliable method of diagnosing or prognosing of fibrosis, hypertrophic scars or keloids.

It is therefore an object of the present invention to provide a method of diagnosis or prognosis of the outcome of the wound or an organ or tissue susceptible to fibrosis.

In a first aspect of the invention, there is provided a method of diagnosis or prognosis of a tissue or organ susceptible of developing fibrosis, such as a lung or skin fibrosis, said method comprising the step of determining the levels of expression of genes encoding different molecular markers in a sample of a tissue or organ from a mammalian, wherein said genes are defined as follows:
- at least one of the following genes show increased expression when compared with the expression in normal fibroblasts of said mammalian:
   EDIL3, EFHD1, FOXS1, HAPLN1, INHBA, KRT16, MICAL2, PI16, POU2F2 and UCN2,
- or at least one of the following genes show decreased expression when compared with the expression in normal fibroblasts of said mammalian:
   APOD, CFB, CXCL1, KIT, MED12L, NR4A3, PTX3, RCAN2, STC1 and TFPI2.

In a preferred embodiment of the invention, a method of diagnosis or prognosis of a wound or skin tissue developing an abnormal scar, such as a fibrosis, a hypertrophic scar or a keloid is provided, said method comprising the step of determining the levels of expression of genes encoding different molecular markers in a sample of a wound or skin from a mammalian, wherein said genes are defined as follows:
- at least one of the following genes show increased expression when compared with the expression in normal dermal fibroblasts of said mammalian:
   EDIL3, CNN1, EFHD1, FOXS1, HAPLN1, KRT16, MICAL2, PI16, TAGLN, POU2F2, and UCN2.
- or at least one of the following genes show decreased expression when compared with the expression in normal dermal fibroblasts of said mammalian:
   APOD, CFB, CXCL1, KIT, MED12L, NR4A3, PTX3, RCAN2, STC1 and TFPI2,

The full identity of the genes according to the invention is available on the NCBI database (http://www.ncbi.nlm.nih.gov/), or is well known to those skilled in the art.

In a preferred aspect of the invention, the tissue or organ is a human tissue or organ, and the normal fibroblasts are Normal Human Fibroblasts.

In a preferred aspect of the invention, the normal fibroblasts arise from the healthy tissue or organ of the said mammalian, and preferably the normal fibroblasts arise from the healthy tissue or organ of the same animal or individual.

As far as the tissue concerned is the skin, the normal dermal fibroblasts arise from the healthy skin of the said mammalian, and preferably the normal dermal fibroblasts arise from the healthy skin of the same animal or individual. In a preferred aspect of the invention, the wound or skin is a human wound or skin, and the normal dermal fibroblasts are Normal Human Dermal Fibroblasts (NHDF).

The term "determining the levels of expression of genes" as used above means qualitative and/or quantitative detection (measuring levels) with reference to a control. Typically the determination of the levels of expression of genes may be measured for example by RT-PCR performed on the sample or in situ hybridization or high-throughput sequencing, such as Lynx Therapeutics' Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope® single molecule sequencing, Single Molecule real time (RNAP), Single Molecule SMRT® sequencing, Nanopore DNA sequencing, VisiGen Biotechnologies approach.

Typically, said determination comprises contacting the sample with selective reagents such as probes, primers or ligands, and thereby detecting the presence, or measuring the amount of nucleic acids of interest originally present in the sample. Contacting may be performed in any suitable device, such as a plate, microtiter dish, test tube, well, glass or column. In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column or a gel. The contacting may be made under any condition suitable for a detectable complex, such as a nucleic acid hybrid, to be formed between the reagent and the nucleic acids of the sample.

In a particular embodiment, the determination of the levels of expression of genes may be determined by quantifying the RNA of said genes. Said RNA are preferably chosen from mRNA and miRNA. Preferably, said RNA are mRNA.

Methods for measuring the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA may be then detected by hybridization (e. g., Northern blot analysis).

Alternatively, the extracted mRNA may be subjected to couple reverse transcription and amplification, such as reverse transcription and amplification by polymerase chain reaction (RT-PCR), using specific oligonucleotide primers that enable amplification of a region in the target gene. Preferably quantitative or semi-quantitative RT-PCR is used. Real-time quantitative or semi-quantitative RT-PCR is particularly advantageous. Extracted mRNA may be reverse-transcripted and amplified, after which amplified sequences may be detected by hybridization with a suitable probe or by direct sequencing, or high-throughput sequencing or any other appropriate method known in the art.

Other methods of amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the RNA of interest herein find utility as hybridization probes or amplification primers. It is understood that such nucleic acids need not be identical, but are typically at least about 80% identical to the homologous region of comparable size, more preferably at least 85% identical and even more preferably at least 90%, preferably at least 95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 3x, 5x or 6x SCC. SCC is a 0.15 M NaCl, 0.015 M Na-citrate).

In the method of the invention, the presence of RNA, preferably total RNA, and more preferably the amount of mRNA, is assayed in the examined samples of wound tissue. All the techniques available for measuring RNA content can be used. Said techniques may include Northern blot, quantitative polymerase chain reaction, NanoString Technologies, microarray technology, or Serial Analysis of Gene expression (SAGE). In the present invention, high-throughput sequencing, such as Lynx Therapeutics' Massively Parallel Signature Sequencing (MPSS), Polony sequencing, 454 pyrosequencing, Illumina (Solexa) sequencing, SOLiD sequencing, Ion semiconductor sequencing, DNA nanoball sequencing, Helioscope® single molecule sequencing, Single Molecule real time (RNAP), Single Molecule SMRT® sequencing, Nanopore DNA sequencing, VisiGen Biotechnologies approach can also be used.

In an alternative embodiment of the invention, the determination of the levels of expression of genes in the sample may also be performed by quantifying the corresponding encoded proteins. All the techniques available for measuring protein content can be used. This may be made by using antibodies.

Such methods comprise contacting a sample with a binding partner capable of selectively interacting with the target protein present in the sample. The binding partner is generally an antibody that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of antibodies against the proteins to be tested. A sample containing or suspected of containing the marker protein is then added to the coated wells. After a period of incubation sufficient to allow the formation of antibody-antigen complexes, the plate(s) can be washed to remove unbound moieties and a detectably labelled secondary binding molecule is added. The secondary binding molecule is allowed to react with any captured sample marker protein, the plate is washed and the presence of the secondary binding molecule is detected using methods well known in the art.

In an another aspect of the invention, there is provided a kit for performing any one or more of the aforementioned methods, wherein said kit comprises probes to detect and quantify the expression level of at least one target gene.

By "probes", it is meant single-stranded nucleic acids of between 10 to 1000 nucleotides in length, for instance of between 10 and 800, more preferably of between 15 and 700, typically of between 20 and 500, which hybridize with the target gene under high stringency hybridization conditions (corresponding to the highest melting temperature Tm, e.g., 50 % formamide, 3x, 5x or 6x SCC. SCC is a 0.15 M NaCl, 0.015 M Na-citrate).

According to a further aspect of the invention, there is provided a kit for performing any one of the aforementioned methods wherein said kit comprises:
(1)A plurality of probes for detecting and quantifying the expression level of all the genes specified in table 1,
(2) Optionally, reagents and instructions pertaining to the use of said probes.

In yet a further preferred aspect of the invention there is provided a kit for determining the prognosis of mammalian wound tissue which comprises:
(1)A plurality of probes for detecting and quantifying the expression level of at least one RNA or protein of each one of the genes of table 1,
(2) Optionally, reagents and instructions pertaining to the use of said probes.

Ideally, the instructions describe how to determine the expression level of each of said genes.

According to a further aspect of the invention there is provided a microarray comprising or consisting of any one or more of the aforementioned sets of probes. The kit according to the invention may use an apparatus such as the Ion Proton Sequencer of Life Technologies, or PGM or MiSeq.

In another aspect of the invention, there is provided a kit for determining wound type in a patient, said kit comprising at least two microarrays, each comprising a plurality of probes for detecting and quantifying the expression level of all the genes specified in one of the above methods.

In a further aspect of the invention, there is provided a method for treating a wound which comprises the step of performing any one or more of the aforementioned methods for determining the classification or prognosis of wound tissue in order to identify whether said wound tissue will develop a fibrosis or become a hypertrophic scar or a keloid or not and selecting an appropriate treatment based on the classification or prognosis of the wound tissue.

In another aspect of the invention, there is provided a therapy consisting in decreasing the expression of PI16 in fibrosis, hypertrophic scar or keloid. Said therapy may consist in the use of an inhibitor of PI16 for treating fibrosis, hypertrophic scar or keloid.

### Role of fibroblasts in wound healing

Fibroblasts are implicated in the process of wound healing, this involves several steps of differentiation from a quiescent fibroblast to a mobilized fibroblast that will transform into a myofibroblast and finally enter apoptosis.

In normal wound healing, fibroblasts get activated, and then differentiate into myofibroblasts presenting contractile properties thanks to their expression of alpha-smooth muscle actin (aSMA). Myofibroblasts are responsible for the deposit of extra cellular matrix and for the wound closure by moving closer the wound edges. In hypertrophic scar, keloid or fibrous wound healing, the activity of myofibroblasts persists and leads to tissue deformation, which is particularly evident, for example, in hypertrophic scars developed after burn injury. The aim of the present invention is to map, at the whole genome scale, the different genes that will be activated or deactivated during this process, and thus providing a molecular signature of abnormal healing leading to abnormal scar or fibrosis

Connective tissues represent a wide variety of physical structures and different functions: tendons, cartilage, bone, dermis, cornea, etc... As organs and tissues have specific functions (for example, skin functions are protection, sensation and heat regulation), connective tissues constituting these tissues and organs have also precise functions provided by specific cell types. For example, in the papillar or reticular dermis collagen I, III and V, XIV, elastic fibers, perlecan or SPARC are found On the contrary, types III, IX, X collagens are found associated with aggregan and dermatan sulfate in tendons.

Fibroblasts are the main cells of connective (or mesenchymal) tissues, in which cells are surrounded by extracellular matrix (contrary to epithelium where they are jointed together). These fibroblasts are active in wound healing of damages organs, as they proliferate, differentiate in myofibroblasts a, secrete collagens and other specific ECM proteins and fibers composing the connective tissue of the organ, leading to the healing and reorganization of the tissue.

Myofibroblasts are defined as the primary source of the excessive ECM proteins deposition occurring during fibrosis. Resident myofibroblasts arise from a population of tissue specific fibroblasts that proliferate and undergo activation in response to injury, as it is the case in many organs such as skin, lungs, or kidney.

The legends of the figures are the following:
Figure 1: levels of αSMA mRNA determined by quantitative RT-PCR
Figure 2: αSMA and tubulin expression determined by Western-Blot
Figure 3A: PI16 mRNA expression (mock siRNA or PI16 siRNA)
Figure 3B: αSMA mRNA expression (mock siRNA or PI16 siRNA)
Figure 4A and B: PI16 mRNA expression at different time points
Table 1: Gene signature list for the fibrosis
Table 2: List of all genes transcripts identified

### Example

In response to a lesion, fibroblasts migrate into the wound where they differentiate into contractile myofibroblasts that will finally enter into apoptosis during the remodeling phase. This differentiation process can be studied ex-vivo in environmentally controlled tissue culture conditions, and therefore the timely controlled succession of different gene expression patterns can be addressed.

### Materials and methods

### Establishment of an ex vivo model for fibrosis or hypertrophic scar

Myofibroblasts represent the key players in the physiological reconstruction of skin after injury and in generating the pathological tissue deformations that characterize fibrosis such as hypertrophic scars (Desmouliere A, Chaponnier C, Gabbiani G (2005) Tissue repair, contraction, and the myofibroblast. Wound Repair Regen 13: 7-12).

To study the myofibroblasts involved in generating hypertrophic or keloid scars, NHDF were cultivated on collagen coated culture plates in DMEM-F12 (Invitrogen), supplemented with 10% FCS (Invitrogen), 5µg/mL of insulin and 1ng/mL of b-FGF (PromoKine) and 10 ng/mL of TGF-β1 (Promocell), as TGF-β1 is known to induce the expression of αSMA in fibroblasts (Desmouliere A, Geinoz A, Gabbiani F, Gabbiani G (1993) Transforming growth factor-beta 1 induces alpha-smooth muscle actin expression in granulation tissue myofibroblasts and in quiescent and growing cultured fibroblasts. J Cell Biol, 1993 jul, 122(1): 103-111).

The efficiency of fibroblast differentiation was estimated by analyzing the expression of the myofibroblast marker alpha smooth muscle actin (aSMA).

This αSMA expression was assessed by RT-qPCR (mRNA levels) and by Western Blot (protein).

### Western Blotting assay

Total proteins were extracted by scratching the cells with lysis buffer (TRIS, NaCl, NP40, EDTA, IMDTT) and incubated 30 min in ice. To remove cell debris, the samples were centrifuged at 13,000 x g for 10 min at 4°C and store at -20°C until use. Protein concentration was determined according to BCA method (Sigma). Equal amounts of total protein (20µg) were loaded to NuPAGE 10% BIS-Tris gel (Invitrogen), separated by migration at 150 V, and transferred to nitrocellulose membrane (Whatman) 1 hour at 30 V. Then, membranes were stained for αSMA (Abcam) and tubulin (Abcam). Incubations were followed by secondary antibodies goat anti-rabbit IgG and goat anti-mouse IgG, respectively, conjugated with horseradish-peroxidase (HRP) (Promega). Signals were detected by ECL chemiluminescence using UptiLight HS WB Substrate (Uptima, Interchim). Bands were digitized with a scanner and the ratio between all bands density of the same blot was calculated by software (ImageJ 1.43u, 64-bit). Relative αSMA expression was normalized to the respective value for tubulin.

### Total RNA Sample Preparation

After four days of experiment, treated fibroblasts were detached with TRIzol Reagent (Invitrogen) and stored at -80°C. Then RNA was purified using chloroform and precipitated by isopropanol. Total RNA was quantified on the NanoDrop 2000c Spectrophotometer (Thermo Scientific). Reverse transcription of 500 ng total RNA to cDNA was done with oligot dT (Invitrogen) using SuperScript III RT (Invitrogen) and RNAse OUT (Invitrogen). The cDNA was store at -20°C.

### Quantitative real-time RT-PCR

Quantitative real-time PCR (RT-qPCR) was done using 5µL of 1:20 diluted cDNA on the LightCycler480 system (Roche) using Maxima SYBR Green qPCR Master Mix (Fermentas). Forward and reverse primers were designed by Eurofins (MWG, αSMA forward: CTGTTTTCCCATCCATTGTG (SEQ ID NO:1), αSMA reverse: CCATGTTCTATCGGGTACTT (SEQ ID NO:2)) and a 100µM stock was stored at -20°C. Forward and reverse primer pairs were used for each RT-qPCR reaction. The cycling conditions were as follows : an initial 95°C for 10 minutes, followed by 45 cycles of 95°C for 15 sec, 58°C for 30 sec, 72°C for 20 sec. LightCycler 480 SW 1.5 was used to evaluate the TM curves, to determine the Cp and to approximate the relative concentration for each amplification reaction.

### Timing of expression

NHDF (Normal Human Dermal Fibroblast) were treated with TGFbeta as described previously for different times (between 1h and 96h). After treatment mRNA were extracted and levels of PI16 mRNA were assessed by RTqPCR.

### siRNA Transfection

The expression of PI16 was knocked down by transiently transfecting human dermal fibroblasts with specific small interfering RNAs (Qiagen). Two different siRNAs were tested. For transfections, fibroblasts were trypsinized and seeded on collagen coated 6-well plates. TGF-β1 was added to the medium as described before. Then, NHDF were treated with 10nM siRNA and 4µL of INTERFERin reagent (PolyPlus), according to the manufacturer's instruction for 6 days. To maintain a sufficient knocking down, a second transfection was performed at 48h. The knockdown of target mRNA was confirmed by RT-qPCR. As a control, mock siRNA (directed against exogenous and non-present GFP mRNA) was used to bypass a possible effect of siRNA transfection into the cells.

### Results

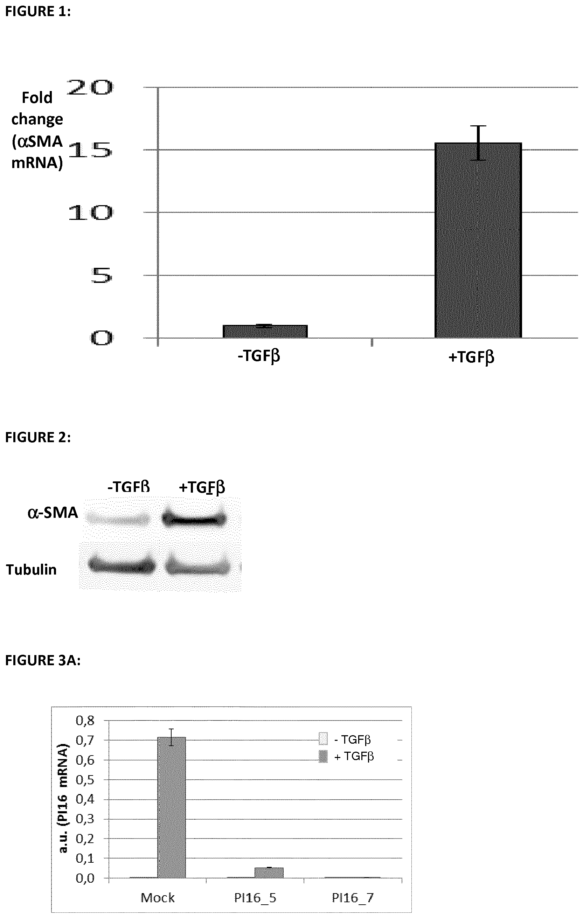

Fibroblasts cultivated with TGF-β in the fibrosis, hypertrophic scar model expressed high level of αSMA (mRNA and protein, figure 1 and 2).

In order to analyze the genes expressed with the fibroblasts in a fibrosis, hypertrophic scar model, mRNA deep sequencing was realized.

Total RNA was extracted by TRIzol Equal amounts of total RNA of the different treated cells (5 to 6 µg) were precipitated by absolute ethanol, supplemented by sodium acetate for RNA sequencing.

The mRNA sequencing was performed by Fasteris SA (Switzerland). RNA was sent as total RNA, after two rounds of polyA purification, the Reverse transcription and the cDNA libraries were done. The sequencing was performed on a HiSeq2000 (Illumina).

One gene can contain different isoforms, and some isoforms can have one or more exons in common. Unfortunately, when the number of reads present in each isoforms is counted and fused in gene entities, sometimes the same reads may be counted several times and thus biases the analyses for genes with numerous isoforms. To solve this problem, it was decided to create a fictive transcript for each gene corresponding to the maximal portion of exon coverage, and to count the number of reads present in these entities. After the normalization and analysis of differential expression steps, only genes showing a differential expression associated with an adjusted p-value of 1.10⁻³ or less were retained. A supplementary filter on the logFC (Fold Change) to study complete lists (the absolute value of logFC has to be superior or equal to 2) was applied.

### Pathologic wound healing analysis: fibrosis, hypertrophic scar or keloid

The aim of the invention was to know if genes are differentially expressed between two conditions, in order to determine if the wound will turn into fibrosis/hypertrophic scar or keloid.

The abundance of gene transcripts between two conditions was compared, normal dermal fibroblasts (cultivated without TGFβ) and myofibroblasts (cultivated with TGFβ) representing the fibrosis or hypertrophic scar situation.

With the p value adjusted and the Log FC filters determined, 171 genes were identified as differentially expressed. Some genes, thanks to their high increased or decreased expression, are of particular interest, for example PI16

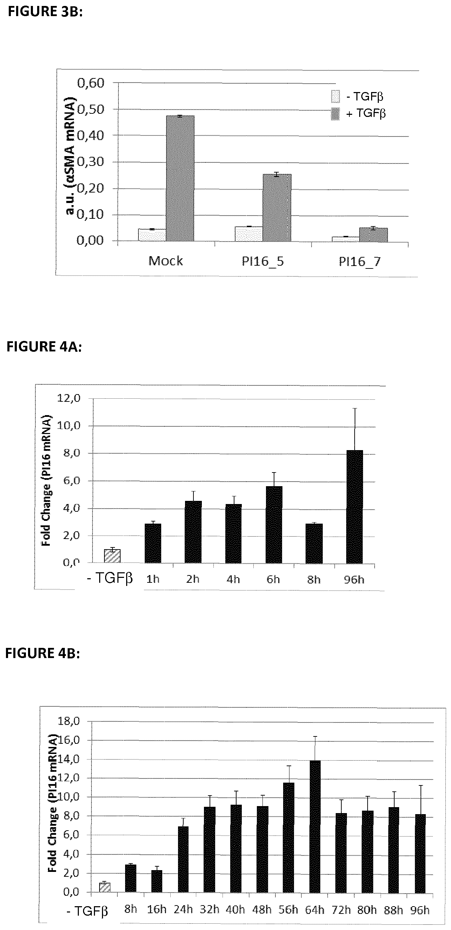

The expression of PI16 is largely increased in fibrosis, hypertrophic scar or keloid model. Whereas PI16 mRNA is usually overexpressed after TGFβ treatment, one can notice here the efficient knockdown of PI16 mRNA levels after siRNA treatment (figure 3A). Very interestingly, when PI16 is down regulated, we can notice a total (for the siRNA PI16_7) or partial (for the siRNA PI16_5) inhibition of the TGFbeta induced-levels of αSMA (fig 3B) mRNA. These differences in the effect of the two siRNA can be correlated with the efficiency of the PI16mRNA knock down. These results are in complete agreement with the expression pattern study of PI16 mRNA after TGFβ treatment. Indeed PI16 mRNA is largely and rapidly up regulated after TGFbeta treatment (x3 after 1h) (Figure 4A ET B)

As a consequence we suggest that the overexpression of PI16 is associated with increase in fibroblast to myofibroblast differentiation, on the contrary a down regulation of PI16 correlates with a non-differentiation behavior of the fibroblast as shown with the siRNA approach.

Thus, PI16 is a favorite candidate for therapy. The present invention is also directed to a therapy consisting in decreasing their expression in fibrosis, hypertrophic scar or keloid.

**Table 1:**

| | |
|---|---|
| APOD | Decrease in fibrosis/hypertrophic scar or keloid |
| CFB | Decrease in fibrosis/hypertrophic scar or keloid |
| CNN1 | Increase in fibrosis/hypertrophic scar or keloid |
| CXCL1 | Decrease in fibrosis/hypertrophic scar or keloid |
| EDIL3 | Increase in fibrosis/hypertrophic scar or keloid |
| EFHD1 | Increase in fibrosis/hypertrophic scar or keloid |
| FOXS1 | Increase in fibrosis/hypertrophic scar or keloid |
| HAPLN1 | Increase in fibrosis/hypertrophic scar or keloid |
| INHBA | Increase in fibrosis/hypertrophic scar or keloid |
| KIT | Decrease in fibrosis/hypertrophic scar or keloid |
| KRT16 | Increase in fibrosis/hypertrophic scar or keloid |
| MICAL2 | Increase in fibrosis/hypertrophic scar or keloid |
| NR4A3 | Decrease in fibrosis/hypertrophic scar or keloid |
| PI16 | Increase in fibrosis/hypertrophic scar or keloid |
| POU2F2 | Increase in fibrosis/hypertrophic scar or keloid |
| PTX3 | Decrease in fibrosis/hypertrophic scar or keloid |
| RCAN2 | Decrease in fibrosis/hypertrophic scar or keloid |
| STC1 | Decrease in fibrosis/hypertrophic scar or keloid |
| TAGLN | Increase in fibrosis/hypertrophic scar or keloid |
| TFPI2 | Decrease in fibrosis/hypertrophic scar or keloid |
| UCN2 | Increase in fibrosis/hypertrophic scar or keloid |

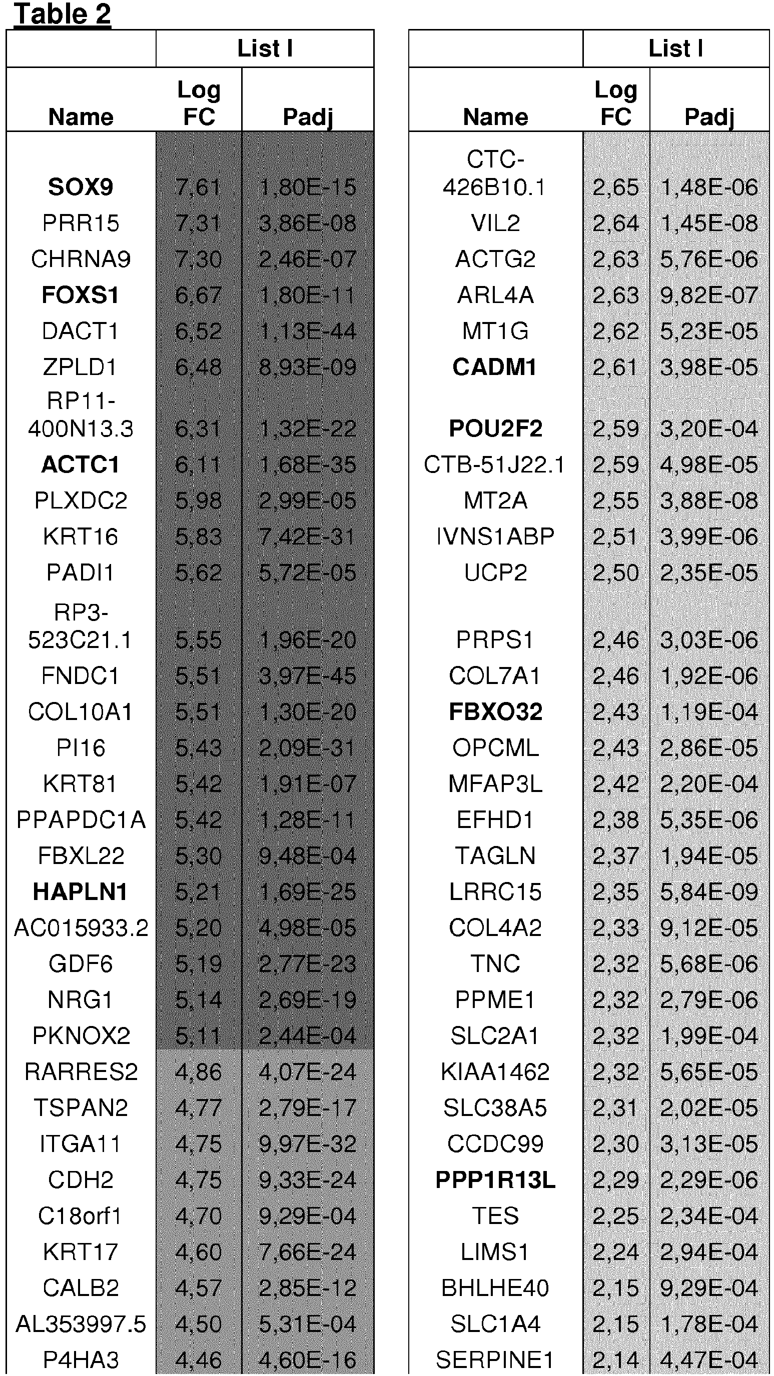

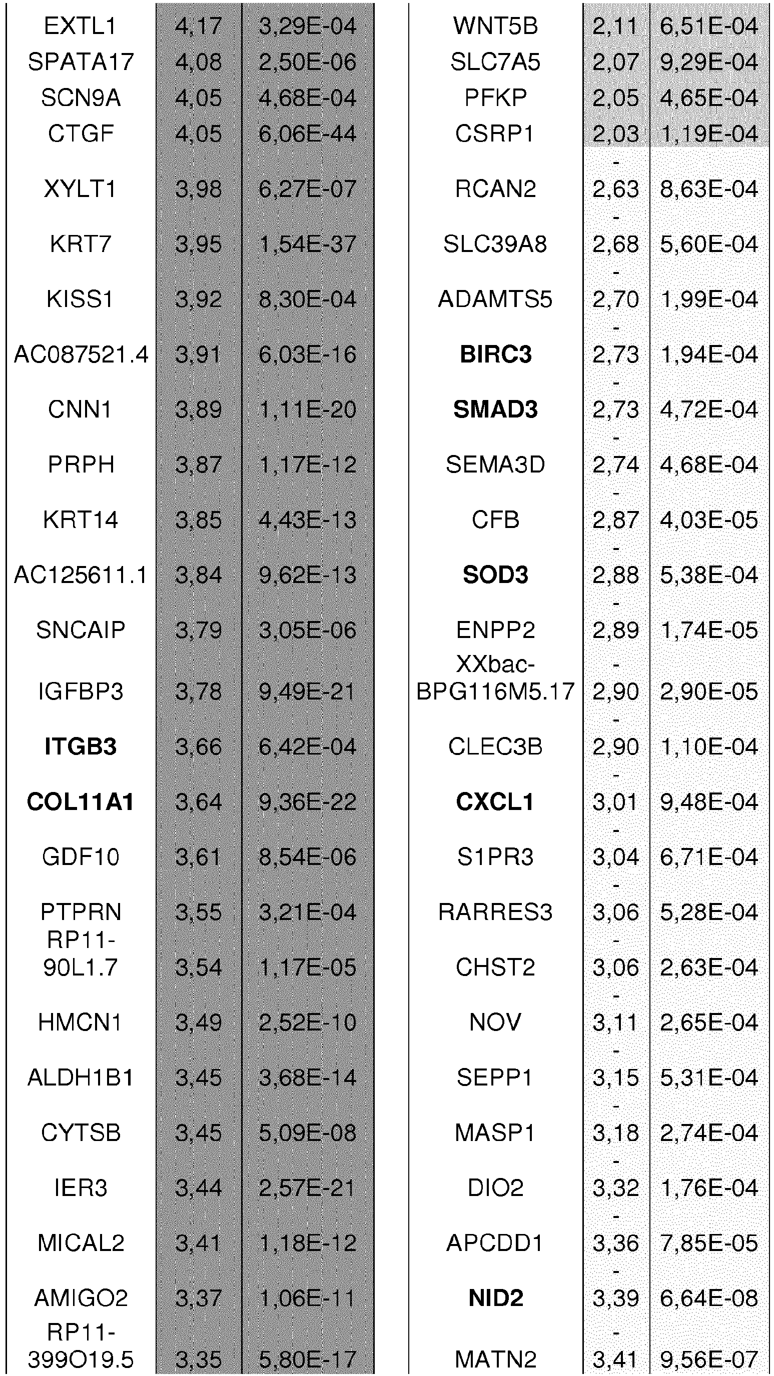

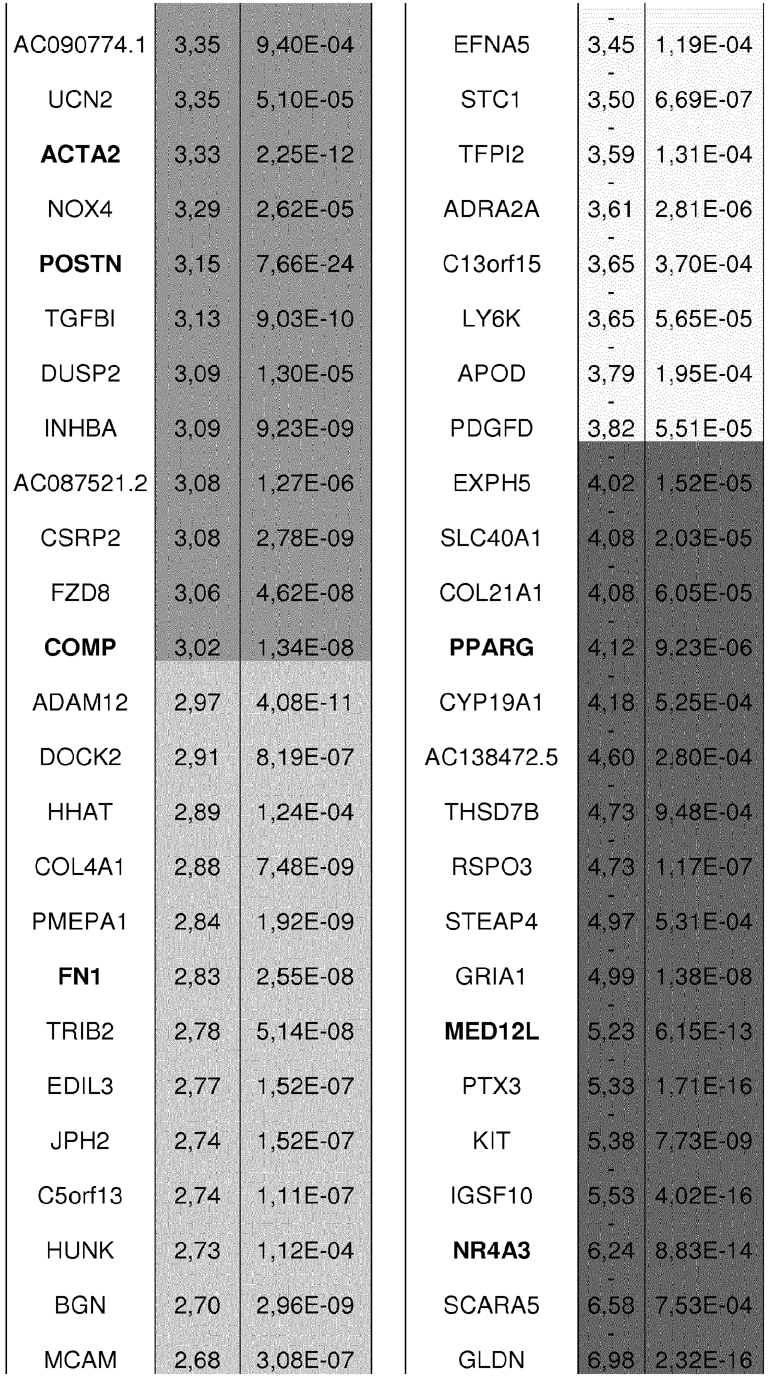

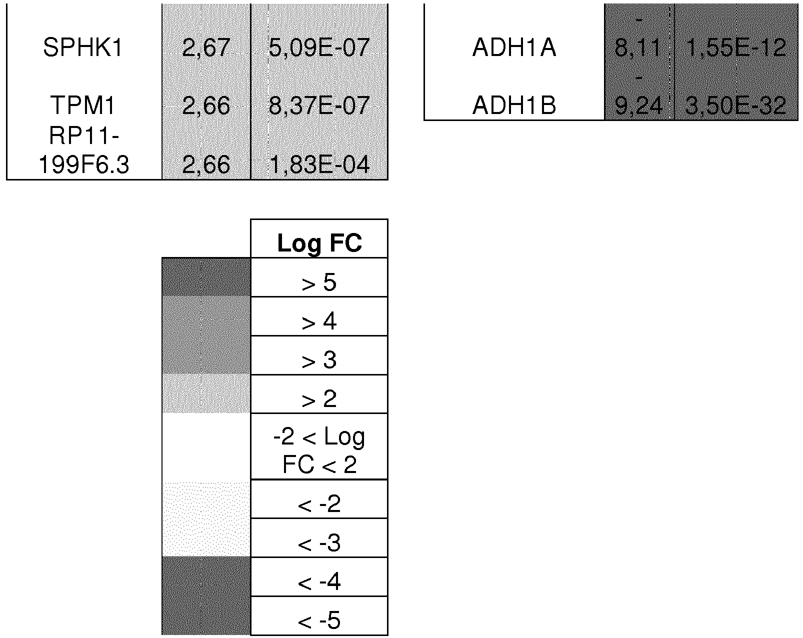

## Claims

1. A method of diagnosis or prognosis of a tissue or organ susceptible of developing a fibrosis, such as a lung or skin fibrosis, comprising the step of determining the levels of expression of genes encoding different molecular markers in a sample of a tissue or organ from a mammalian, wherein said genes are defined as follows :
- at least PI16 gene shows increased expression when compared with the expression in normal fibroblasts of said mammalian.

2. A method of diagnosis or prognosis according to claim 1 wherein said tissue is skin susceptible of developing an abnormal scar, such as a skin fibrosis, a hypertrophic scar or a keloid, comprising the step of determining the levels of expression or genes encoding different molecular markers in a sample of a wound from a mammalian, wherein said genes are defined as follows :
- at least one of the following genes show increased expression when compared with the expression in normal dermal fibroblasts of said mammalian:
EDIL3, CNN1, EFHD1, FOXS1, HAPLN1, KRT16, MICAL2, TAGLN, POU2F2 and UCN2,
- or at least one of the following genes show decreased expression when compared with the expression in normal dermal fibroblasts of said mammalian:
APOD, CFB, CXCL1, KIT, MED12L, NR4A3, PTX3, RCAN2, STC1 and TFPI2.

3. A method according to any preceding claims, wherein said wound tissue is human wound tissue, and normal dermal fibroblasts are Normal Human Dermal Fibroblasts (NHDF).

4. A method according to any preceding claims, wherein the normal dermal fibroblasts arise from the healthy skin of the said mammalian and more preferably the wound tissue and the normal dermal fibroblasts arise from the same animal or individual.

5. A method according to any preceding claims, wherein the said levels of expression of genes are determined by quantifying the corresponding RNA.

6. A method according to claim 5, wherein said RNA is chosen from mRNA and miRNA.

7. A method according to any one of claims 1-6, wherein the said levels of expression of genes are determined by quantifying the corresponding encoded proteins.

8. A method according to claim 7, wherein said proteins are measured by using antibodies.

9. A kit for performing any one or more of the aforementioned methods according to any one of claims 1-8, wherein said kit comprises:
(1) A plurality of probes for detecting and quantifying the expression levels of all the genes specified in table 1,
(2) Optionally, reagents and instructions pertaining to the use of said probes.

10. A kit for determining the prognosis of mammalian wound which comprises:
(1) A plurality of probes for detecting and quantifying the expression level of at least one RNA or protein of each one of the genes of table 1,
(2) Optionally, reagents and instructions pertaining to the use of said probes.

11. A microarray consisting of any one or more of the sets of probes in claims 9-10.

12. A kit for determining a wound type in a patient, comprising:
at least two microarrays comprising a plurality of probes for detecting and
quantifying the expression levels of all the genes specified in any one of claims 1 or 2.
